(19) Europäisches Patentamt
European Patent Office
Office européen des brevets

(11) Publication number: **0 185 092 B1**

(12) **EUROPEAN PATENT SPECIFICATION**

(45) Date of publication of patent specification: **07.10.92**

(51) Int. Cl.[5]: **C12P 19/32**, C12N 15/52, //(C12P19/32,C12R1:19)

(21) Application number: **85901555.4**

(22) Date of filing: **12.03.85**

(86) International application number: **PCT/JP85/00122**

(87) International publication number: **WO 85/04187 (26.09.85 85/21)**

# (54) PROCESS FOR PREPARING 5'-GUANYLIC ACID.

(30) Priority: **12.03.84 JP 46682/84**
**20.04.84 JP 78673/84**

(43) Date of publication of application: **25.06.86 Bulletin 86/26**

(45) Publication of the grant of the patent: **07.10.92 Bulletin 92/41**

(84) Designated Contracting States: **DE FR GB**

(56) References cited:
**JP-B-57 166 992**
**JP-B-58 111 697**
**JP-B-58 175 492**

**CHEMICAL ABSTRACTS, vol. 87, no. 19, 7th November 1977, page 287, abstract no. 148547a, Columbus, Ohio, US; Y. FUKUMAKI et al.: "Expression of a bacterial gene for guanine synthesis inserted into colicin E1 factor by an in vitro recombination", & BIOCHIM. BIOPHYS. ACTA 1977, 478(1), 90-8**

(73) Proprietor: **KYOWA HAKKO KOGYO CO., LTD.**
**6-1, Ohtemachi 1-chome**
**Chiyoda-ku Tokyo-to(JP)**

(72) Inventor: **FUJIO, Tatsuro**
**6-29-1, Sagamidai Sagamihara-shi**
**Kanagawa 228(JP)**
Inventor: **MARUYAMA, Akihiko**
**4-19-23, Eifuku Suginami-ku**
**Tokyo 168(JP)**
Inventor: **NISHI, Tatsunari**
**5-19-24, Okura Setagaya-ku**
**Tokyo 157(JP)**
Inventor: **OZAKI, Akio**
**3-9-10, Naka-machi Machida-shi**
**Tokyo 194(JP)**
Inventor: **ITO, Seiga**
**218-14, Aihara Sagamihara-shi**
**Kanagawa 229(JP)**
Inventor: **OZAKI, Atsuko**
**3-9-10, Naka-machi Machida-shi**
**Tokyo 194(JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

**CHEMICAL ABSTRACTS, vol. 94, no. 13, 30th March 1981, page 581, abstract no. 101345n, Columbus, Ohio, US; & JP-A-80 124 495 (AJINOMOTO CO., INC.) 25-09-1980**

**CHEMICAL ABSTRACTS, vol. 100, no. 26, 25th June 1984, page 402, abstract no. 21562h, Columbus, Ohio, US; & JP-A-58 175 492 (AJINOMOTO CO., INC.) 14-10-1983**

**CHEMICAL ABSTRACTS, vol. 101, no. 1, 7th January 1984, page 149, abstract no. 84912p, Columbus, Ohio, US; M.S. THOMAS et al.: "Molecular cloning and characterization of the gua regulatory region of Escherichia coli K12", & MGG, MOL. GEN. GENET. 1984, 195(1-2), 238-45**

**CHEMICAL ABSTRACTS, vol. 82, no. 17, 28th April 1975, page 377, abstract no. 110311c, Columbus, Ohio, US; & JP-A-74 27 356 (YAMASA SHOYU CO., LTD) 17-04-1974**

(74) Representative: **Vossius & Partner**
**Siebertstrasse 4 P.O. Box 86 07 67**
**W-8000 München 86(DE)**

## Description

The present invention relates to a process for producing 5'-guanylic acid (hereinafter referred to as GMP). More particularly, it relates to a process for producing GMP by using Escherichia coli having an ability to convert 5'-xanthylic acid (hereinafter referred to as XMP) and ammonia and/or L-glutamine to GMP and also having an ability to convert adenosine monophosphate (hereinafter referred to as AMP) to adenosine triphosphate (hereinafter referred to as ATP) in the presence of an energy donor other than phosphorylated compounds, and to a process for producing GMP from XMP by culturing in a medium a transformant obtained by transforming a microorganism by means of a recombinant DNA of a DNA fragment containing a gene of guanylic acid synthetase capable of converting XMP and ammonia and/or L-glutamine to GMP in the presence of ATP (the guanylic acid synthetase is also called xanthylic acid aminase and will be hereinafter referred to as GMP synthetase) and a vector DNA fragment and carrying out reaction using the resulting culture liquor, cells or their treated products as an enzyme source.

GMP is in a great demand as a seasoning agent, and the development of a process for producing GMP at lower cost has been desired. Processes for producing GMP so far known include (1) a process by enzymatically decomposing ribonucleic acid, (2) a process by chemically phosphorylating guanosine, (3) a process by converting XMP to GMP by means of bacteria belonging to the genus Brevibacterium or the genus Corynebacterium [Japanese Patent Publication No. 39069/71; Japanese Patent Application No. 187050/82], etc. Process (3) is an advantageous process using a microorganism capable of reproducing ATP necessary for the conversion from AMP by assimilating cheap energy donors such as glucose, as illustrated below.

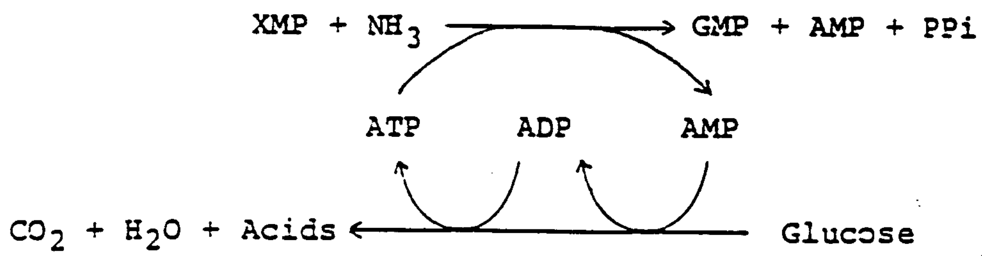


Furthermore, a process using a mutant strain whose GMP synthetase activity is intensified by a resistance to chemicals is known [Biotechnol. Bioengineer., 13, 229-240 (1971)].

As a result of studies to develop an advantageous process for producing GMP, it has been found that GMP can be produced with Escherichia coli having a GMP synthetase activity and an ability to convert AMP to ATP by utilizing an energy donor other than phosphorylated compounds in the presence of phosphate ions and magnesium ions (the ability will be hereinafter referred to as ATP reproduction ability), and that a transformant having a high activity of converting XMP to GMP can be obtained by cloning a gene of GMP synthetase (hereinafter sometimes referred to as guaA) into plasmid vector pBR322, ligating a promoter of tryptophan operon of Escherichia coli (hereinafter referred to as trp promoter or Ptrp) to the upstream side of guaA to construct a recombinant plasmid, and transforming a microorganism with the resulting plasmid.

According to the present invention, XMP and ammonia and/or L-glutamine are converted to GMP in an aqueous medium in the presence of cells of a microorganism selected from

the group consisting of Escherichia coli K294/pXA1, FERM BP-498, Escherichia coli K294/pXA10, FERM BP-499 and Escherichia coli K294/pXAR33, FERM BP-500, and in the presence of an energy donor other than phosphorylated compounds, and GMP is recovered from the reaction solution, whereby GMP can be produced in high yield.

Further Escherichia coli (hereinafter sometimes referred to as E. coli) which can be used in the present invention since it has an ability to form GMP from said substrate are E. coli PL1068 [see J. Gen. Microbiol., 123, 27-37 (1981)] and E. coli K294 ($r^-$, $m^+$) FERM BP-526.

The transformant to be used in the present invention can be obtained in the following manner.

The DNA fragment containing a GMP synthetase gene to be used in the present invention includes those derived from procaryotes, bacteriophage and plasmid, among which DNA fragments containing a GMP synthetase gene and derived from Escherichia coli and plasmids containing said DNA fragment are preferred. Specifically, pLC34-10 or pLC32-25, a hybrid plasmid of a DNA fragment derived from the

chromosome of Escherichia coli and containing both a gene of inosinic acid dehydrogenase (hereinafter sometimes referred to as guaB) and guaA with DNA of colicin $E_1$ (hereinafter referred to as Col$E_1$) is a preferable guaA source [both are disclosed in Methods in Enzymology 68, 396-408 (1979)], and plasmid DNA is separated from Escherichia coli JA200 strain containing these plasmids [obtained from Yale university, E. coli Genetic Stock Center (hereinafter referred to as CGSC), disclosed in L. Clarke and J. Carbon, Cell 9, 91-99 (1976)) and purified according to a known method [Nucleic Acids Research 7, 1513 (1979), this method is hereinafter used for the recovery of plasmids].

On the chromosome of Escherichia coli, guaA is positioned just at the downstream side of guaB, and both guaA and guaB genes form one operon together with the promoter operator region at the upstream side of guaB [Molec. gen. Genet. 147, 203-208 (1976)].

When GMP is accumulated in the cells, the expression of both genes is suppressed. Furthermore, it is known that there is a secondary promoter between guaB and guaA (or in the guaB region) which has a lower transcription activity than the promoter at the upstream side [J. Bact. 131, 685-688 (1977)].

Any plasmid can be used for the construction of a recombinant plasmid capable of efficiently expressing the GMP synthetase to be used in the present invention, so far as it can allow the gene of GMP synthetase to be expressed in Escherichia coli. Those having a property of giving such properties as a resistance to antibiotics, etc. to a host microorganism are more preferable. Specific examples thereof include pBR322 [Gene. 2, 95 (1977)] and pBR325 [Gene. 4, 121 (1978)], and more preferably, vectors pGBK3, pGBY1, etc. having a trp promoter and used in Examples of the present invention can be mentioned.

Preparation of the recombinant of the DNA fragment containing a gene and the vector DNA can be carried out according to a known in vitro recombinant DNA technique. The in vitro DNA recombination is usually carried out by physical or enzymatic cleavage and ligation (ligase reaction) of a donor DNA containing a desired gene and a vector DNA. Recovery of the desired recombinant from the ligase reaction solution can be attained by directly transforming Escherichia coli with the DNA mixture, selectively separating the transformant endowed with a genetic character derived from the genetic information of the desired gene, and isolating the recombinant from the cultured cells of the transformant by extraction. When a vector capable of giving a resistance to chemicals to a host strain such as pBR322, etc. is used, the desired recombinant can also be obtained by first selecting a chemical-resistant strain after the transformation, and then separating the transformant endowed with the genetic character derived from the genetic information of the desired gene.

Any microorganism can be used as a host microorganism according to the present invention, so far as it can express the recombinant DNA and can be utilized to increase the production of GMP, and strains belonging to Escherichia coli and having an ability to incorporate DNA are preferable. Specifically, Escherichia coli PL1068 (guaA$^-$) [see J. Gen. Microbiol. 126, 497-501 (1981)], Escherichia coli K294 ($r^-$, $m^+$) FERM BP-526, etc. can be used.

Transformation of a host microorganism with the recombinant DNA can be carried out according to a known method [Cohen et al.: Proc. Natl. Acad. Sci. U.S.A., 69, 2110 (1972), the transformation of E. coli is hereinafter carried out according to this procedure]. Recombinants capable of expressing guaA possessed by the recombinant plasmid in the host cells can be selected by using as a host E. coli PL1068 strains, which are guaA-deficient strains, and selecting the strains which are guaA$^+$ after the transformation.

Culturing of Escherichia coli is carried out in an ordinary medium containing a carbon source, a nitrogen source, inorganic matters, amino acids, vitamins, etc. under aerobic conditions.

As a carbon source, carbohydrates such as glucose, fructose, sucrose, maltose, mannitol, sorbitol, etc., sugar alcohol, glycerol, starch hydrolyzate solution, molasses, etc. can be used. Furthermore, various organic acids such as pyruvic acid, lactic acid, citric acid, etc., and various amino acids such as glutamic acid, methionine, etc. can be used.

As a nitrogen source, ammonia, various inorganic and organic ammonium salts such as ammonium chloride, ammonium sulfate, ammonium carbonate, ammonium acetate, etc., amino acids such as glutamic acid, glutamine, methionine, etc., or nitrogen-containing organic materials such as peptone, NZ-amine, corn steep liquor, meat extract, yeast extract, casein hydrolyzate, fish meal or its digest, chrysalis hydrolyzate, etc. can be used.

As an inorganic matter, potassium dihydrogen phosphate, sodium monohydrogen phosphate, magnesium sulfate, sodium chloride, calcium chloride, iron chloride, copper sulfate, manganese chloride, ammonium molybdate, zinc sulfate, etc. are added, if necessary. It is not necessary to add vitamines, amino acids, etc. required for the growth of the microorganism to the medium if they are supplied together with other medium components described above.

Culturing is carried out at a temperature of 20 to 50 °C, preferably 28 to 42 °C and a pH around

neutrality under aerobic conditions by shaking culture or aerated stirring culture. The culturing is usually complete in 1 to 24 hours.

The culture of the microorganism can be used as such, and also treated products of the culture, for example, a concentrate or dried product of the culture, a filtrate or cells obtained by centrifuging the culture, dried cells, acetone-treated cells, surfactant-treated cells, organic solvent-treated cells, lytic enzyme-treated cells, immobilized cells, extracted enzyme preparation from the cells, etc. can be used.

The contact reaction producing GMP can be carried out in any aqueous medium. Most preferably, XMP and ammonia or glutamine, and if necessary, ATP, an energy donor, phosphate ions, magnesium ions, a surfactant, an organic solvent, etc. are subjected to reaction in the culture liquor of a microorganism, whereby GMP is accumulated in the culture liquor, or the said components are added to the culture liquor, cells or their treated products after the completion of culturing, and subjected to reaction at 20 to 50 °C for 1 to 48 hours, whereby GMP can be accumulated. In that case, it is desirable to adjust the pH to 6-10. As an ammonium source, an ammonium salt is usually used. Concentrations (g/ℓ) of the substrates and additives in the medium or reaction solution are 1-100 XMP, 1-100 ATP, 1-50 $MgSO_4 \cdot 7H_2O$, 1-25 $(NH_4)_2SO_4 \cdot 7H_2O$, and 1-25 glutamine.

As an XMP source, any of those containing XMP, such as a microbial XMP fermentation liquor as such, a concentrate thereof, its partially purified preparation, etc. can be used besides the highly purified preparation, as long as it does not inhibit the formation of GMP.

As an energy donor, any of carbohydrates such as glucose, arabinose, lactose, maltose, sucrose, mannitol, sorbitol, trehalose, molasses, starch hydrolyzate, etc.; organic acids such as pyruvic acid, lactic acid, acetic acid, α-ketoglutaric acid, etc.; amino acids such as glycine, alanine, aspartic acid, glutamic acid, etc. can be used, as long as it is a non-phosphorylated compound and can be utilized by the Escherichia coli used. These are used at a concentration of 1-200 g/ℓ.

It is desirable to keep the concentration of phosphate ions and magnesium ions in a range of 4-400 mM in the contact reaction solution. When the amount of the ions taken into the reaction solution from the culture liquor or cells falls within the said range of concentration, it is not necessary to add the ions thereto, whereas when the ions are insufficient, they are added so that the amount will fall within the said range of concentration. As phosphate ions, any of sodium salt, potassium salt, magnesium salt, etc. of phosphoric acid can be used. As magnesium ions, any of inorganic salts and organic acid salts can be used.

As a surfactant, cationic surfactants such as polyoxyethylene stearylamine (e.g. Nymeen S-215, made by Nippon Oil and Fats Co., Ltd.), cetyl-trimethylammonium bromide, etc.; anionic surfactants such as sodium oleylamide sulfate, etc., non-ionic surfactants such as polyoxyethylene sorbitan monostearate (e.g. Nonion ST221, made by Nippon Oil and Fats Co., Ltd.), etc., amphoteric surfactants such as laurylbetaine (e.g. Anon BF, made by Nippon Oil and Fats Co., Ltd.), etc. can be used. They are usually used at a concentration of 0.1 to 50 g/ℓ, preferably 1 to 20 g/ℓ.

As an organic solvent, toluene, xylene, analiphatic alcohol, benzene, ethyl acetate, etc. can be used at a concentration of 0.1 to 50 mℓ/ℓ, preferably 1 to 20 mℓ/ℓ.

GMP accumulated in the aqueous reaction solution can be recovered according to the ordinary procedure using activated carbon, ion exchange resin, etc.

## Brief Description of the Drawings

Fig. 1 shows a process for constructing plasmid pXAR33.
Fig. 2 shows a process for constructing plasmid pGC7.
Fig. 3 shows a process for constructing plasmid pGKA2.
Fig. 4 shows a process for constructing plasmid pGBK3.
Fig. 5 shows a process for constructing plasmid pGBY1.

## Best Mode for Carrying Out the Invention

### Example 1

Construction of a recombinant plasmid which efficiently expresses GMP synthetase

1) Subcloning of guaA into pBR322:

E. coli JA200 strain carrying pLC34-10, hybrid plasmid of gua operon (containing guaA and guaB) derived from the chromosome of E. coli and $ColE_1$ was inoculated in L medium containing 10 g/ℓ Bacto-Tryptone (made by Difco Co.), 5 g/ℓ yeast extract (made by Difco Co.) and 5 g/ℓ sodium chloride and adjusted to pH 7.2, and cultured at 30°C for 18 hours. Plasmid pLC34-10 was separated from the cultured cells and purified according to the known procedure described above. pBR322 used as a vector was separated from E. coli JA194 strain which carries it [B. Ratzlein and J. Carbon, Proc. Natl. Acad. Sci. U.S.A. 74, 487 (1977)] and purified in the same manner as above. For the culturing and preservation of microorganisms described hereinafter, L medium was used unless otherwise specified.

pLC34-10, which has a size of about 15 kilobases (which will be hereinafter abbreviated as Kb), was cleaved with restriction enzyme EcoRI at one position and with PstI at three positions (see Fig. 1). It was expected that there was guaA on the EcoRI-PstI fragment of about 7 kb containing one PstI cleavage site [Biochem. Biophys. Res. Commun., 72, 1129-1136 (1976)].

Then, 5µg of pLC34-10 plasmid DNA prepared above was dissolved in 50 µℓ of a buffer solution containing 10 mM Trishydrochloric acid (pH 7.5), 50 mM NaCl, 7 mM $MgCl_2$ and 6 mM 2-mercaptoethanol (hereinafter referred to as "Y-50 buffer solution"), and 20 units of restriction enzyme EcoRI [made by Takara Shuzo Co., the restriction enzymes hereinafter are all products of Takara Shuzo Co. unless otherwise specified] was added thereto. The mixture was subjected to digestion reaction at 37°C for 2 hours, and then 5 units of PstI (made by New England Biolabs Co.) was added thereto. The mixture was subjected to partial digestion reaction at 37°C for 30 minutes, and the reaction was discontinued by heat treatment at 65°C for 10 minutes. A plasmid DNA fragment of about 7 Kb was purified from the digest by low-gelling-temperature agarose gel electrophoresis [Analytical Biochemistry, 98, 305 (1979)] (this procedure was hereinafter used for the purification of DNA fragments). Separately, 2 µg of pBR322 plasmid DNA was dissolved in 20 µℓ of Y-50 buffer solution, and 8 units of EcoRI and 8 units of PstI were added thereto. The mixture was subjected to digestion reaction at 37°C for 2 hours, and the larger plasmid fragment (about 3.6 Kb) was purified.

Then, about 0.2 µg of the DNA fragment derived from pLC34-10 and about 0.05 µg of the DNA fragment derived from pBR322 which were obtained above were treated with 2 units of T4 ligase at 4°C for 18 hours in 40 µℓ of a buffer solution containing 20 mM Tris-hydrochloric acid (pH 7.6), 10 mM $MgCℓ_2$, 10 mM dithiothreitol and 0.5 mM ATP (hereinafter referred to as "T4 DNA ligase buffer solution"). E. coli PL1068 strain mutated at guaA was transformed with the thus obtained recombinant plasmid DNA according to the procedure of Cohen, et al. described above, whereby a transformant having a resistance to tetracycline (20 µg/mℓ) and being devoid of the guanine requirement shown by the host was obtained.

A plasmid was separated and purified from the transformant, and structural analysis of the plasmid was made by digesting the DNA with restriction enzymes such as EcoRI, PstI, etc. As a result, it was found to be a recombinant plasmid wherein the EcoRI-PstI DNA fragment (about 7 Kb) derived from pLC34-10 was inserted at the EcoRI-PstI site of pBR322, and named pXA1. E. coli KLC421 ($guaA^-$, $guaB^-$) [J. Bact. 139, 320 (1979)] transformed with pXA1 was smeared onto M9 plate medium (containing 1g of $NH_4Cℓ$, 6g of $Na_2HPO_4$, 3g of $KH_2PO_4$, 5g of NaCl, 0.25g of $MgSO_4 \cdot 7H_2O$, 3g of glucose, 4g of vitamin $B_1$, and 2g of Casamino acid in 1ℓ of water, and also 1.5% agar) to investigate the growth. It was found that it grew on the plate medium containing 5 mg/ℓ xanthine or guanine, whereas no growth was observed on the M9 plate medium containing 5 mg/ℓ hypoxanthine. This result shows that pXA1 carries only guaA derived from pLC34-10 though pLC34-10 carries both guaA and guaB. The strain obtained by transforming E. coli K294 with pXA1 was deposited with the Fermentation Research Institute, Agency of Industrial Science and Technology as E. coli K294/pXA1 FERM BP-498 on March 8, 1984.

2) Ligation of trp promoter (hereinafter referred to as Ptrp) to the upstream side of guaA:

First, 3 µg of pXA1 DNA was dissolved in 30 µℓ of Y-50 buffer solution, and 15 units of HindIII was added thereto. The mixture was subjected to digestion reaction at 37°C for 2 hours, and 2 µℓ of 2M NaCℓ and 15 units of MluI were added thereto. The mixture was subjected to digestion reaction at 37°C for 2 hours. After heat treatment at 65°C for 10 minutes, the smaller DNA fragment containing guaA (about 3.3 Kb) was purified. On the other hand, pGBK3 was used as a plasmid containing Ptrp. A procedure for constructing pGBK3 is shown in Reference Example 2. pGBK3 has a HindIII cleavage site at the downstream side of Ptrp (see Fig. 1).

3 µg of pGBK3 DNA was digested with HindIII and MluI in the same manner as above, and the larger DNA fragment containing Ptrp (about 4 Kb) was purified. Then, about 0.2 µg of the DNA fragment derived from pXA1 and about 0.1 µg of the DNA fragment derived from pGBK3 which were obtained above were subjected to ligation reaction at 4°C for 18 hours in 20 µℓ of T4DNA ligase buffer solution in the presence of one unit of T4DNA ligase. E. coli K294 strain was transformed with the thus obtained recombinant plasmid, whereby a transformant having a resistance to ampicillin was obtained. A plasmid DNA was

separated and purified from the transformant, and by structural analysis, it was confirmed that the plasmid had the structure wherein the DNA fragment containing guaA and derived from pXA1 was inserted at the downstream side of Ptrp derived from pGBK3. The plasmid was named pXA10 (see Fig. 1). E. coli strain carrying pXA10 was deposited with the Fermentation Research Institute as E. coli K294/pXA10 FERM BP-499 on March 8, 1984.

3) Shortening of the distance between Ptrp and guaA:

As described later, no substantial difference was recognized in the expression of guaA between the strain carrying pXA10 and the strain carrying pXA1 (see Table 1).

Thus, plasmid pXAR33 having a shortened distance between Ptrp and guaA was constructed in the following manner (see Fig. 1).

First, about 10 μg of pXA10 DNA was dissolved in 60 μℓ of a buffer solution containing 10 mM Tris-hydrochloric acid (pH 7.5), 150 mM NaCℓ, 7 mM $MgCℓ_2$ and 2-mercaptoethanol (hereinafter referred to as "Y-150 buffer solution"), and 40 units of HindIII were added thereto. The mixture was subjected to digestion reaction at 37°C for 2 hours. To 30 μℓ of the HindIII-digested reaction solution were added 20 μℓ of Ba131 buffer solution at 5 fold concentration [100 mM Tris•HCl (pH 8.1), 60 mM $MgCℓ_2$, 60 mM $CaCℓ_2$ and 3 M NaCℓ], 46 μℓ distilled water and 2 units of Ba131 (made by Bethesda Research Laboratories), and the mixture was subjected to digestion reaction at 30°C. During the period of 5 to 50 minutes after the start of reaction, the reaction solution was sampled in portions of 10 μℓ from time to time, and each portion was put into 20 μℓ of a phenol : chloroform mixture (1:1 by volume). The mixture was thoroughly stirred to discontinue the reaction, and was ice-cooled. After centrifuging, an upper layer was sampled, and the 2-fold volume of ice-cooled ethanol was added thereto. The mixture was allowed to stand at -80°C for 30 minutes. After the respective ethanol mixtures were centrifuged, the supernatant was discarded, while 5 μℓ of Y-150 buffer solution at a 10-fold concentration and 43 μℓ of distilled water were added to the precipitates to dissolve the precipitates. Then, 3 units of MluI was added to the respective solutions, and the solutions were subjected to digestion reaction at 37°C for 2 hours. The reaction solutions were heat-treated at 65°C for 10 minutes, and the smaller DNA fragments (not more than 3 Kb) were purified. On the other hand, plasmid pGBY1 in which the NruI site on the DNA fragment derived from pBR322 of pGBK3 was changed to BglII site was used as a vector (the procedure for constructing pGBY1 is shown in Reference Example 3).

3 μg of pGBY1 DNA was dissolved in 20 μℓ of Y-50 buffer solution, and 15 units of HindIII was added thereto. The mixture was subjected to digestion reaction at 37°C for 2 hours. After the reaction, the reaction solution was subjected to extraction with phenol and chloroform, and to precipitation with ethanol. The DNA fragment was dissolved in 40 μℓ (total volume) of 50 mM Tris-hydrochloric acid (pH 7.6), 7 mM $MgCℓ_2$, 6 mM 2-mercaptoethanol, 0.25 mM dATP, 0.25 mM dGTP, 0.25 mM dTTP and 0.25 mM dCTP, and then 6 units of E. coli DNA polymerase I•Klenow fragment (1 μℓ, made by New England Biolabs) was added thereto. The mixture was subjected to reaction at 15°C for 2 hours, and the 5'-protruding end formed by HindIII digestion was changed to a blunt end.

After the extraction with phenol-chloroform and the precipitation with ethanol, the precipitate of DNA was dissolved in 20 μℓ of Y-150 buffer solution, and 15 units of MluI was added thereto. The mixture was subjected to digestion reaction at 37°C for 2 hours. The larger DNA fragment having Ptrp (about 4.2 Kb) was purified from the MluI-digestion reaction product by low-gelling-temperature agarose gel electrophoresis.

To about 0.1 μg each of the thus obtained DNA fragments derived from pXA10 was added 0.05 μg of the DNA fragment derived from pGBY1. The respective mixtures were subjected to ligation reaction at 4°C for 18 hours in 20 μℓ (total volume) of T4 DNA ligase buffer solution in the presence of one unit of T4 DNA ligase. Then, E. coli K294 strains were transformed with the thus obtained recombinant plasmid DNA, and the transformants having a resistance to ampicillin were selected. The obtained ampicillin-resistant strains were cultured in M9 liquid medium (M9 plate medium freed from agar) at 30°C for 18 hours, and the GMP synthetase activity was investigated in the manner described below to select strains having a higher activity than that of E. coli K294 strain carrying pXA10. Plasmid DNAs were separated from the strains having a higher activity (R-33 strain) and purified, and the structures thereof were analyzed. It was found that plasmid pXAR33 possessed by the R-33 strain had the HindIII-MluI DNA fragment containing guaA of pXA10. Among the analyzed plasmid DNAs, pXAR33 had the shortest DNA fragment, and the R-33 strain which carried it showed a high GMP synthetase activity. The R-33 strain was deposited with the Fermentation Research Institute as Escherichia coli K294/pXAR33 FERM BP-500 on March 8, 1984.

4) GMP synthetase activity of the strains carrying recombinant plasmids:

Determination of GMP synthetase activity was carried out according to a known procedure [J. Biol. Chem., 226, 351-363 (1957)] after modification as described below.

Seed culture of E. coli for the activity determination test was inoculated in M9 liquid medium and subjected to shaking culture at 30 °C for 18 hours. The culture liquor was diluted with distilled water or concentrated by suspending in an appropriate amount of distilled water after centrifugation, and then toluene was added thereto to make a final concentration of 20 mℓ/ℓ. The mixture was shaken at 37 °C for 20 minutes.

The toluene-treated culture liquor was put into a reacting solution comprising 160 mM Tris-hydrochloric acid (pH 8.6), 12 mM ATP, 25 mM XMP, 16 mM $MgSO_4 \cdot 7H_2O$ and 40 mM $(NH_4)_2SO_4$, and the mixture was shaken at 42 °C to conduct conversion of XMP to GMP.

GMP formed was quantitatively determined by sampling the reaction solution from time to time, mixing the sample with 40-fold volume of 3.5% perchloric acid, centrifuging the mixture, and measuring the absorbance of the supernatant at 290 nm.

In Table 1, the GMP synthetase activities of the strains used or obtained in the present invention are shown, wherein one unit of activity is defined as an amount of activity which forms 1 μmole of GMP per minute.

Table 1

| Host E. coli strain | Plasmid | Deposit No. | Activity per wet cell (unit/g wet cell) |
|---|---|---|---|
| K294 | - | FERM BP-526 | 0.88 |
| K294 | pXA1 | FERM BP-498 | 17.08 |
| K294 | pXA10 | FERM BP-499 | 16.91 |
| K294 | pXAR33 | FERM BP-500 | 73.50 |

Example 2

The culture liquor (wet cell weight: 4.6 mg/mℓ) of pXAR33-carrying strain (R-33) obtained in Example 1 was concentrated 40-fold by centrifugation, and toluene was added thereto to make a final concentration of 20 mℓ/ℓ. The mixture was shaken at 37 °C for 20 minutes. Then, 30 mℓ of a reaction solution comprising 20 mg/mℓ $XMP \cdot Na_2 \cdot 7H_2O$, 20 mg/mℓ $ATP \cdot Na_2 \cdot 3H_2O$ and 10 mg/mℓ $(NH_4)_2SO_4$ and adjusted to pH 8.6 was placed in a 200 mℓ-beaker, and 0.2 mℓ of said cell suspension containing toluene was added thereto. The reaction mixture was stirred by means of a magnetic stirrer (900 rpm), and kept at 42 °C for 5 hours, while adjusting the pH to 8.6 with caustic soda. As a result of the reaction, 13.0 mg/mℓ 5'-$GMP \cdot Na_2 \cdot 7H_2O$ was formed in the reaction solution. When the host K294 strain was used in place of the R-33 strain, the yield was less than 1 mg/mℓ.

Example 3

R-33 strain was inoculated in a 300 mℓ-Erlenmeyer flask containing 30 mℓ of L medium and subjected to shaking culture at 30 °C for 18 hours by means of a rotary shaker (220 rpm). To the culture liquor were added toluene, $XMP \cdot Na_2 \cdot 7H_2O$, $ATP \cdot Na_2 \cdot 3H_2O$ and $(NH_4)_2SO_4$ to make 20 μℓ/mℓ, 20 mg/mℓ, 20 mg/mℓ and 10 mg/mℓ, respectively. Shaking culture was continued for additional 10 hours while keeping the temperature at 42 °C and adjusting the pH to 8.6 with caustic soda. As a result, 13.7 mg/mℓ $GMP \cdot Na_2 \cdot 7H_2O$ was formed and accumulated in the medium. When the host K294 strain was used in place of the R-33 strain, the yield was less than 1 mg/mℓ.

Example 4

E. coli K294 (FERM BP-526) strain was inoculated in a 1ℓ-Erlenmeyer flask containing 200 mℓ of M9 medium comprising 6 g/ℓ disodium phosphate, 3 g/ℓ potassium dihydrogen phosphate, 5 g/ℓ sodium chloride, 1 g/ℓ ammonium chloride, 4 mg/ℓ thiamine hydrochloride, 250 mg/ℓ magnesium sulfate and 3 g/ℓ glucose, and subjected to reciprocating shaking culture at 28 °C overnight. The cells were collected by centrifugation, and preserved in a frozen state (-20 °C).

Water was added to the frozen cells at room temperature to make a suspension at final concentration of 200 g/ℓ as wet cell weight, and 40 g/ℓ $XMP \cdot Na_2 \cdot 7H_2O$, 50 g/ℓ glucose, 2 g/ℓ sodium phytate, 5 g/ℓ $Na_2HPO_4$ and 5 g/ℓ $MgSO_4 \cdot 7H_2O$ were dissolved in the suspension (concentrations are final ones). The resulting mixture was put into 200 mℓ-beakers in portions of 20 mℓ. The respective beakers were kept at 37°C in a thermostat water bath and stirred at 900 rpm by means of magnetic stirrers for 24 hours to convert XMP to GMP, while adjusting the pH to 7.4 with 9% aqueous ammonia (see Table 2). In Table 2, (1) shows the result obtained by using the reaction mixture of said composition as such and (2) shows the result obtained by using the reaction mixture of said composition further admixed with 4 g/ℓ Nymeen S-215 and with 10 mℓ/ℓ xylene.

Table 2

| | Nymeen S-215 and xylene | $GMP \cdot Na_2 \cdot 7H_2O$ (g/ℓ) |
|---|---|---|
| (1) | - | 2.2 |
| (2) | + | 5.2 |

Example 5

E. coli K294 strain and E. coli K294/pXA10 strain were cultured in the same manner as in Example 4 except that M9 medium containing 50 mg/ℓ ampicillin was used for the culturing of K294/pXA10 strain. As a result of reaction under the same conditions as in Example 4-(2) using Nymeen S-215 and xylene, the E. coli K294 strain formed 5.5 g/ℓ $GMP \cdot Na_2 \cdot 7H_2O$ in 23 hours, and the E. coli K294/pXA10 strain formed 23.8 g/ℓ $GMP \cdot Na_2 \cdot 7H_2O$ in 6 hours.

Example 6

E. coli K294/pXA10 strain was cultured in M9 medium containing 50 mg/ℓ ampicillin in the same manner as in Example 4. The amount of cells was 7.5 g/ℓ as wet cell weight. To the culture liquor were added XMP, glucose, sodium phytate, $Na_2HPO_4$, $MgSO_4 \cdot 7H_2O$, Nymeen S-215 and xylene to make a final concentration of 40 g/l, 50 g/l, 2 g/l, 5 g/l, 5 g/l,4 g/l and 10 mℓ/ℓ, respectively, and conversion of XMP to GMP was conducted in the same manner as in Example 4. As a result, 5.2 g/ℓ $GMP \cdot Na_2 \cdot 7H_2O$ were formed in 23 hours.

Reference Example 1

Construction of recombinant plasmid pGKA2 which expresses human interferon (IFN)-$\gamma$ (see also J. Biochem. 97(1985)153-159) (see Figs. 2 and 3):

(a) Insertion of human IFN-$\gamma$ DNA into the expression vector pKYP11:

In this example, 6 $\mu$g of plasmid pIFN$\gamma$-G4 which was isolated from ATCC 39123 strain by the above-described method for isolation of a plasmid was dissolved in 50 $\mu$ℓ (total volume) of a solution containing 20 mM Tris-hydrochloric acid (Tris-HCℓ) (pH 7.5), 10 mM $MgC\ell_2$, 10 mM dithiothreitol and 50 mM NaCℓ. Then, 12 units each of restriction enzymes PvuII and HindIII were added and digestion reaction was carried out at 37°C for 4 hours. The reaction solution was heated at 65°C for 7 minutes to inactivate the enzymes and subjected to purification by low-gelling-temperature agarose gel electrophoresis to obtain 1.2 $\mu$g of a DNA fragment containing human IFN-$\gamma$ DNA of 1.3 Kb.

Separately, 4 $\mu$g of pKYP11 was dissolved in 40 $\mu$ℓ (total volume) of a solution containing 20 mM Tris-HCℓ (pH 7.5), 10 mM $MgC\ell_2$, 10 mM dithiothreitol and 50 mM NaCℓ. 3 units of BamHI was added and digestion reaction was carried out at 37°C for 3 hours. The reaction solution was heated at 65°C for 5 minutes to inactivate the enzyme. Thereafter, 30 $\mu$M each of dATP, dCTP, dGTP and dTTP were added and 8 units of Escherichia coli DNA polymerase I (Klenow fragment, product of New England Biolabs, 1 $\mu$ℓ) was added. Fill-in reaction was carried out at 15°C for 1 hour and the reaction solution was heated at 68°C for 15 minutes to inactivate the DNA polymerase I. 10 units of HindIII was added and digestion reaction was carried out at 37°C for 3 hours, followed by heating at 65°C for 5 minutes to inactivate the HindIII.

The digestion reaction solution of the plasmid pKYP-11 thus obtained was subjected to purification by low-gelling-temperature agarose gel electrophoresis to obtain about 2.5 µg of a DNA fragment of about 4.7 Kb containing Ptrp.

Then, 0.5 µg of the DNA fragment of 1.3 Kb containing human IFN-γ DNA and 1.0 µg of the DNA fragment of about 4.7 Kb containing Ptrp, which was obtained from the plasmid pKYP11, were dissolved in 20 µℓ of a solution containing 20 mM Tris-HCℓ (pH 7.5), 6 mM $MgC\ell_2$, 5 mM dithiothreitol and 500 µM ATP, and 4 units of T4 DNA ligase (product of New England Biolabs) was added. Ligation reaction was carried out at 4°C for 18 hours, and Escherichia coli HB101 was transformed with the obtained recombinant plasmid mixture by conventional technique to obtain an $Ap^R$ colony. A plasmid, pGC7 illustrated in Fig. 2 was separated from the culture broth of the colony. The structure of pGC7 was confirmed by digestion with HindIII, BamHI, HpaI, SalI, EcoRI and ClaI and agarose gel electrophoresis. Escherichia coli strain carrying pGC7 has been deposited with the Fermentation Research Institute as Escherichia coli IGC7 (FERM BP-497).

(b) Construction of recombinant plasmid pGKA2:

In this example, 6 µg of pGC7 DNA obtained in Reference Example 1(a) was dissolved in 50 µℓ (total volume) of a solution containing 20 mM Tris-HCℓ (pH 7.5), 10 mM $MgC\ell_2$, 10 mM dithiothreitol and 10 mM NaCℓ, and 12 units of restriction enzyme BstNI (product of New England Biolabs) was added. Reaction was carried out at 60°C for 3 hours. Then, 150 mM NaCℓ and 8 units of SalI were added and digestion reaction was carried out at 37°C for 3 hours. The reaction solution was again heated at 65°C for 5 minutes to inactivate the SalI and subjected to purification by low-gelling-temperature agarose gel electrophoresis to obtain about 0.8 µg of a DNA fragment of about 1,125 bp containing a large portion of the human IFN-γ DNA.

Separately, 3 µg of pKYP10 was dissolved in 40 µℓ (total volume) of a solution containing 20 mM Tris-HCℓ (pH 7.5), 10 mM $MgC\ell_2$, 10 mM dithiothreitol and 100 mM NaCℓ. 6 units each of restriction enzymes HindIII and SalI were added and digestion reaction was carried out at 37°C for 3 hours. The reaction solution was heated at 65°C for 5 minutes to inactivate HindIII and SalI and subjected to purification by low-gelling-temperature agarose gel electrophoresis to obtain about 1.8 µg of a DNA fragment of about 4.1 Kb containing Ptrp.

The N-terminal amino acid of the mature human IFN-γ polypeptide is Cys. In order to express mature IFN-γ DNA, it is necessary to furnish an initiation codon (ATG) just before the 5'-terminal codon TGT (Cys) and further to adjust the length between SD-sequence at the downstream side of Ptrp and ATG to a suitable length of 6-18 bp. Therefore, the following DNA linker was synthesized.

```
HindIII      MetCysTyrCys      BstNI
5' AGCTT ATG TGTTACTGCC 3'        (18-mer)
       3' ATACACAATGACGGT 5'      (15-mer)
```

Two single chain DNAs of 18-mer and 15-mer were synthesized by a conventional tri-ester method [R. Crea, et al.: Proc. Natl. Acad. Sci., 75, 5765 (1978)]. Then, 2 µg each of the 18-mer and 15-mer DNAs were dissolved in 20 µℓ (total volume) of a solution containing 50 mM Tris-HCℓ (pH 7.5), 10 mM $MgC\ell_2$, 5 mM dithiothreitol, 0.1 mM EDTA and 1 mM ATP. 30 units of T4 polynucleotide kinase (product of Boehringer Mannheim) was added and phosphorylation reaction was carried out at 37°C for 60 minutes.

Then, 2 µg each of phosphorylated 18-mer and 15-mer DNAs were mixed and the mixture was heated at 70°C for 5 minutes and allowed to stand at room temperature for annealing to obtain the DNA linker having the structure given above.

0.4 µg of the BstNI - SalI fragment of 1,125 bp obtained above and derived from pGC7 and 1.0 µg of the DNA fragment of 4.1 Kb obtained by digestion of the expression vector pKYP10 with HindIII and SalI were dissolved in 25 µℓ (total volume) of a solution containing 20 mM Tris-HCℓ (pH 7.5), 6 mM $MgC\ell_2$, 5 mM dithiothreitol and 500 µM ATP. About 0.1 µg of the DNA linker mentioned above was added to the mixture, followed by addition of 6 units of T4 DNA ligase. Ligation reaction was carried out at 4°C for 17 hours. Escherichia coli HB101 was transformed using the obtained recombinant plasmid mixture by conventional technique to obtain an $Ap^R$ colony. A plasmid, pGKA2 illustrated in Fig. 3 was isolated from the

culture broth of the colony. The structure of pGKA2 was confirmed by digestion with EcoRI, ClaI, HindIII, BstNI and SalI and agarose gel electrophoresis. It was confirmed by the method of Maxam-Gilbert [A.M. Maxam, et al.: Proc. Natl. Acad. Sci., 74, 560 (1977)] that the base sequence from the SD-sequence (AAGG) to the initiation codon (ATG) in the plasmid pGKA2 was "AAGGGTATCGATAAGCTTATG".

The human IFN-γ DNA in pGKA2 is different from known DNAs in that the DNA has RsaI site and the ninth amino acid of the human IFN-γ polypeptide encoded by the DNA is glutamine (Gln).

Further, the synthesized DNA used above is different from the one used by P.W. Gray, et al. having the following structure:

met cys tyr cys
AATTC ATG TGT TAT TGT C
GTACACAATAACAGT

in the underlined parts. Thus, pGKA2 has a restriction site for BstNI, CCAGG, in the DNA region coding for human IFN-γ and this feature also distinguishes pGKA2 from known plasmids. Furthermore, the length and structure between the SD-sequence and ATG are important because of influence on the expression of proteins in *Escherichia coli*. The base sequence between the SD-sequence and ATG in pGKA2 is apparently different from that in the known recombinant plasmid pIFN-γ trp48 (P.W. Gray, et al.)

*Escherichia coli* carrying pGKA2 has been deposited with the Fermentation Research Institute as *Escherichia coli* IGKA2 (FERM BP-496).

## Reference Example 2

Construction of recombinant plasmid pGBK3 which expresses human IFN-γ under control of tacI promoter:

As the first step for constructing the recombinant plasmid, insertion of the transcription termination site of *Escherichia coli* lipoprotein (lpp) gene (hereinafter referred to as lpp terminator) into IFN-γ-expressing plasmid pGKA2 (the procedure for constructing pGKA2 is shown in Reference Example 1) was carried out according to the following procedures (a), (b), (c) and (d) (see Fig. 4).

(a) Construction of pGBDl:

In this example, 2 μg of plasmid pIFNγ-G4 (about 3.6 Kb) was dissolved in 20 μℓ of Y-50 buffer solution, and 6 units of PvuII was added thereto. Digestion reaction was carried out at 37 °C for 2 hours, and then discontinued by heat treatment at 65 °C for 10 minutes. Then, 0.1 μg of the digest was subjected to ligation reaction at 4 °C for 18 hours with 2 units of T4 DNA ligase in 20 μℓ of T4 DNA ligase buffer solution in the presence of 5 picomoles of 5'-phosphorylated BamHI linker (5'-pCCGGATCCGG-3'; made by Collaborative Research Co.).

*Escherichia coli* HB101 strain was transformed with the thus obtained recombinant plasmid DNA to obtain an Ap-resistant colony. A plasmid DNA was isolated from the transformant, and the DNA was digested with restriction enzymes such as BamHI, etc. to conduct structural analysis of the plasmid. As a result, it was confirmed that recombinant plasmid pGBDl wherein BamHI linker was inserted at the PvuII site of pIFNγ-G4 was obtained.

(b) Construction of pKYP14:

Construction of recombinant plasmid pKYP14 used as a source of the lpp terminator is described below.

First, 5 μg of trp promoter-carrying plasmid pKYP10 (Japanese Unexamined Published Patent Application No. 110600/83) was dissolved in 40 μℓ of Y-100 buffer solution, and 10 units of BamHI was added thereto. Digestion reaction was carried out at 37 °C for 2 hours, and then 1 μℓ of Y-100 buffer solution, 2.5 μℓ of 1M NaCℓ, 5.5 μℓ of distilled water and 20 units of SaℓI were added thereto. The reaction was further carried out at 37 °C for 2 hours. After heat treatment at 65 °C for 10 minutes, the larger plasmid DNA fragment (about 4.9 Kb) was purified by low-gelling-temperature agarose gel electrophoresis. Separately, 5 μg of lpp terminator-carrying plasmid pIN-II-AI [K. Nakamura, et al.: The EMBO Journal 1, 771 (1982)] (the

same as pKEN045 in Japanese unexamined Published Patent Application No. 140800/82) was digested with BamHI and Saℓl in the same manner as above. The resulting BamHI-Saℓl fragment of about 0.95 Kb containing the lpp terminator was purified.

Then, about 0.1 µg of the DNA fragment derived from pKYP10 and about 0.05 µg of the DNA fragment derived from pIN-II-Al which were obtained above were subjected to ligation reaction at 4°C for 18 hours in 20 µℓ of T4 DNA ligase buffer solution in the presence of one unit of T4 DNA ligase.

A plasmid DNA was isolated from the Escherichia coli HB101 strain transformed with the thus obtained recombinant plasmid DNA, and subjected to structural analysis, whereby it was confirmed that the lpp terminator was inserted at the downstream side of plasmid pKYP14 possessed by the IKYP14 strain.

(c) Construction of pGBJ2:

Insertion of lpp terminator at the downstream side of IFN-γDNA was carried out by recombining recombinant plasmids pGBD1 and pKYP14 obtained in the foregoing procedures (a) and (b) in the following manner.

First, 5 µg of plasmid pGBD1 (about 3.6 Kb) was dissolved in 30 µℓ of a buffer solution containing 10 mM Tris-HCl (pH 7.5), 7 mM $MgCl_2$ and 6 mM 2-mercaptoethanol (hereinafter referred to as "Y-0 buffer solution"), and 10 units of ClaI was added thereto. Digestion reaction was carried out at 37°C for two hours. After heat treatment at 65°C for 10 minutes and subsequent ice cooling, 2 µℓ of Y-0 buffer solution at 10-fold concentration, 5 µℓ of 1M NaCℓ, 12 µℓ of distilled water and 2.0 units of restriction enzyme BamHI were added thereto and mixed. Digestion reaction was carried out at 37°C for 2 hours. The plasmid DNA was partially digested with BamHI during the reaction. The resulting ClaI-BamHI DNA fragment (about 1.3 Kb) containing IFN-γ DNA was purified. Separately, 5 µg of plasmid pKYP14 (about 5.8 Kb) containing lpp terminator was digested with 10 units of ClaI and 20 units of BamHI in 50 µℓ of Y-50 buffer solution for 2 hours, and then the larger plasmid DNA fragment of about 5.0 Kb containing the lpp terminator was purified. The thus obtained DNA fragment derived from pKYP14 (about 0.1 µg) and DNA fragment derived from pGBDl (about 0.05 µg) were subjected to ligation reaction at 4°C for 18 hours in 20 µℓ of T4 DNA ligase buffer solution in the presence of one unit of T4 DNA ligase.

A plasmid DNA was isolated from the Escherichia coli HB101 strain transformed with the thus obtained recombinant plasmid, and subjected to structural analysis, whereby it was confirmed that plasmid pGBJ2 possessed by the IGBJ2 strain had the structure wherein the lpp terminator was inserted at the downstream side of IFN-γDNA.

(d) Construction of pGBK3:

Plasmid pGBK3 having the structure wherein the lpp terminator is inserted at the downstream side of IFN-γ DNA was constructed by recombining recombinant plasmid pGBJ2 obtained in the foregoing step (c) and IFN-γ-expressing plasmid pGKA2 (see Reference Example 1) in the following manner.

First, about 5 µg of plasmid pGKA2 (about 5.2 Kb) was dissolved in 30 µℓ of Y-50 buffer solution, and more than 10 units of PstI was added thereto. Digestion reaction was carried out at 37°C for 2 hours. After heat treatment at 65°C for 10 minutes and subsequent ice cooling, 2 µℓ of Y-150 buffer solution at 10-fold concentration, 3 µℓ of 1M NaCl, 14 µℓ of distilled water and 10 units of restriction enzyme NcoI (made by New England Biolabs, the same restriction enzyme was used hereinafter) were added thereto, and digestion reaction was carried out at 37°C for 2 hours. After heat treatment at 65°C for 10 minutes, a plasmid DNA fragment of about 1.85 Kb containing IFN-γ DNA was purified. Then, about 5 µg of recombinant plasmid pGBJ2 (about 6.4 Kb) obtained above was subjected to the same treatment as applied to pGKA2, and the resulting PstI-NcoI plasmid DNA fragment of about 4.7 Kb was purified. The thus obtained DNA fragment derived from pGKA2 (about 0.1 µg) and DNA fragment derived from pGBJ2 (about 0.1 µg) were subjected to ligation reaction at 4°C for 18 hours in 20 µℓ of T4 DNA ligase buffer solution in the presence of one unit of T4 DNA ligase. A plasmid DNA was separated and purified from the Escherichia coli HB101 strain transformed with the thus obtained recombinant plasmid, and subjected to structural analysis, whereby it was confirmed that plasmid pGBK3 possessed by the IGBK3 strain had the desired structure.

Reference Example 3

Construction of pGBYl (see Fig. 5):

Recombinant plasmid pGBYl wherein BglII linker is inserted at the NruI site of recombinant plasmid pGBK3 obtained in Reference Example 2 (d) was obtained in the following manner.

First, about 2 μg of plasmid pGBK3 (about 5.9 Kb) was dissolved in 20 μℓ of Y-100 buffer solution, and 4 units of NruI (made by New England Biolabs) was added thereto. Digestion reaction was carried out at 37 °C for 2 hours, and discontinued by heat treatment at 65 °C for 10 minutes. Then, 0.1 μg of the digest was subjected to ligation reaction at 4 °C for 18 hours with 2 units of T4 DNA ligase in 20 μℓ of T4 DNA ligase buffer solution in the presence of 5 pM of 5'-phosphorylated BglII linker (5' pCAGATCTG-3'; made by Takara Shuzo Co.).

Escherichia coli HB101 strain was transformed with the thus obtained recombinant plasmid DNA to obtain an Ap-resistant colony. A plasmid DNA was isolated from the tranformant, and the structural analysis of the plasmid was carried out by digesting the DNA with restriction enzymes such as BglII, etc., whereby it was confirmed that recombinant plasmid pGBYI wherein BglII linker was inserted at the NruI site of pGBK3 was obtained.

## Claims

**1.** A process for producing 5'-guanylic acid (GMP), which comprises the steps of (i) and (ii):
(i) converting 5'-xanthylic acid (XMP), and ammonia and/or L-glutamine to GMP in an aqueous solution in the presence of (a) cells of a microorganism selected from the group consisting of Escherichia coli K294/pXA1, FERM BP-498, Escherichia coli K294/pXA10, FERM BP-499 and Escherichia coli K294/pXAR33, FERM BP-500, and (b) an energy donor other than phosphorylated compounds; and
(ii) recover GMP from the reaction solution.

**2.** A process according to Claim 1, wherein the conversion reaction is carried out in the presence of at least one member selected from phosphate ions, magnesium ions, a surfactant and an organic solvent.

**3.** The process according to Claim 1, wherein the energy donor is selected from the group consisting of carbohydrates, organic acids and amino acids.

**4.** Escherichia coli K294/pXA1, FERM BP-498.

**5.** Escherichia coli K294/pXA10, FERM BP-499.

**6.** Escherichia coli K294/pXAR33, FERM BP-500.

## Patentansprüche

**1.** Verfahren zur Erzeugung von 5'-Guanylsäure (GMP), umfassend die Schritte (i) und (ii):
(i) Umwandlung von 5'-Xanthylsäure (XMP) und Ammoniak und/oder L-Glutamin zu GMP in einer wäßrigen Lösung in Gegenwart von (a) Zellen eines Mikroorganismus ausgewählt aus der Gruppe Escherichia coli K294/pXA1, FERM BP-498, Escherichia coli K294/pXA10, FERM BP-499, und Escherichia coli K294/pXAR33, FERM BP-500, und (b) einem anderen Energiedonor als phosphorylierten Verbindungen;und
(ii) Gewinnung von GMP aus der Reaktionslösung.

**2.** Verfahren nach Anspruch 1, wobei die Umwandlungsreaktion in Gegenwart von mindestens einem der folgenden Produkte durchgeführt wird: Phosphationen, Magnesiumionen, ein grenzflächenaktives Mittel und ein organisches Lösungsmittel.

**3.** Verfahren nach Anspruch 1, wobei der Energiedonor ausgewählt ist aus der Gruppe Kohlenhydrate, organische Säuren und Aminosäuren.

**4.** Escherichia coli K294/pXA1, FERM BP-498.

**5.** Escherichia coli K 294/pXA10, FERM BP-499.

**6.** Escherichia coli K294/pXAR33, FERM BP-500.

## Revendications

1. Procédé de production de l'acide 5'-guanylique (GMP), comprenant les étapes (i) et (ii) :
   (i) conversion de l'acide 5'-xanthylique (XMP), et de l'ammoniac et/ou de la L-glutamine, en GMP, dans une solution aqueuse, en présence (a) de cellules d'un microorganisme choisi dans le groupe constitué par Escherichia coli K294/pXA1, FERM BP-498, Escherichia coli K294/pXA10, FERM BP-499 et Escherichia coli K294/pXAR33, FERM BP-500, et (b) d'un donneur d'énergie autre que des composés phosphorylés ; et
   (ii) récupération du GMP dans la solution réactionnelle.

2. Procédé conforme à la revendication 1, dans lequel la réaction de conversion est effectuée en présence d'au moins un composé choisi parmi les ions phosphate, les ions magnésium, un tensioactif et un solvant organique.

3. Procédé conforme à la revendication 1, dans lequel le donneur d'énergie est choisi dans le groupe constitué par les hydrates de carbone, les acides organiques et les acides aminés.

4. Escherichia coli K294/pXA1, FERM BP-498.

5. Escherichia coli K294/pXA10, FERM BP-499.

6. Escherichia coli K294/pXAR33, FERM BP-500.

Fig. 1

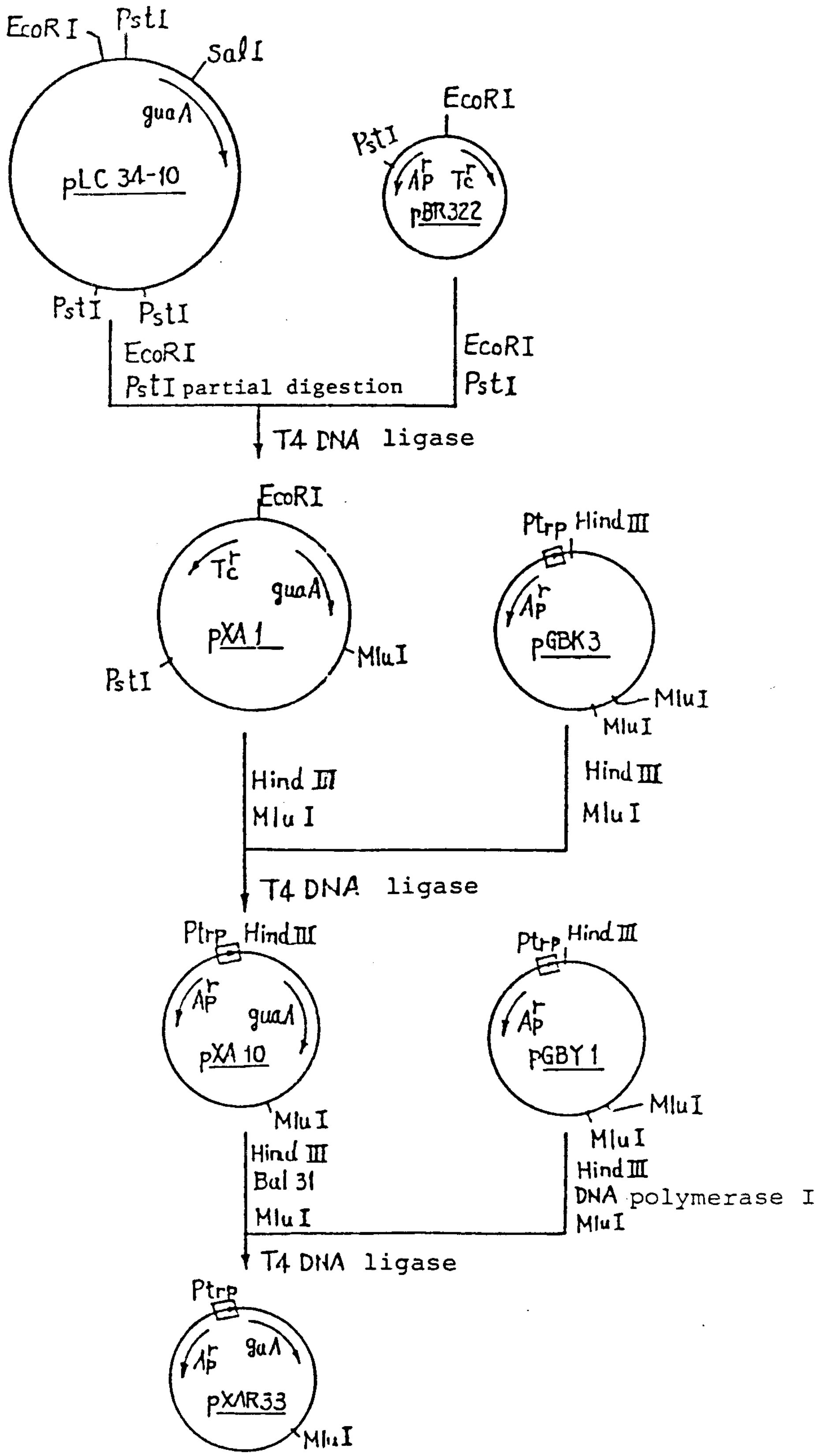
EcoR I
PstI
SalI
guaA
pLC 31-10
PstI
PstI
EcoRI
PstI
Ap[r]
Tc[r]
pBR322
EcoRI
PstI partial digestion
EcoRI
PstI
T4 DNA ligase
EcoRI
Tc[r]
guaA
pXA1
MluI
PstI
Ptrp
HindIII
Ap[r]
pGBK3
MluI
MluI
Hind III
Mlu I
Hind III
Mlu I
T4 DNA ligase
Ptrp
HindIII
Ap[r]
guaA
pXA10
MluI
Ptrp
HindIII
Ap[r]
pGBY1
MluI
MluI
Hind III
Bal 31
MluI
MluI
HindIII
DNA polymerase I
MluI
T4 DNA ligase
Ptrp
Ap[r]
guA
pXAR33
MluI

F i g . 2

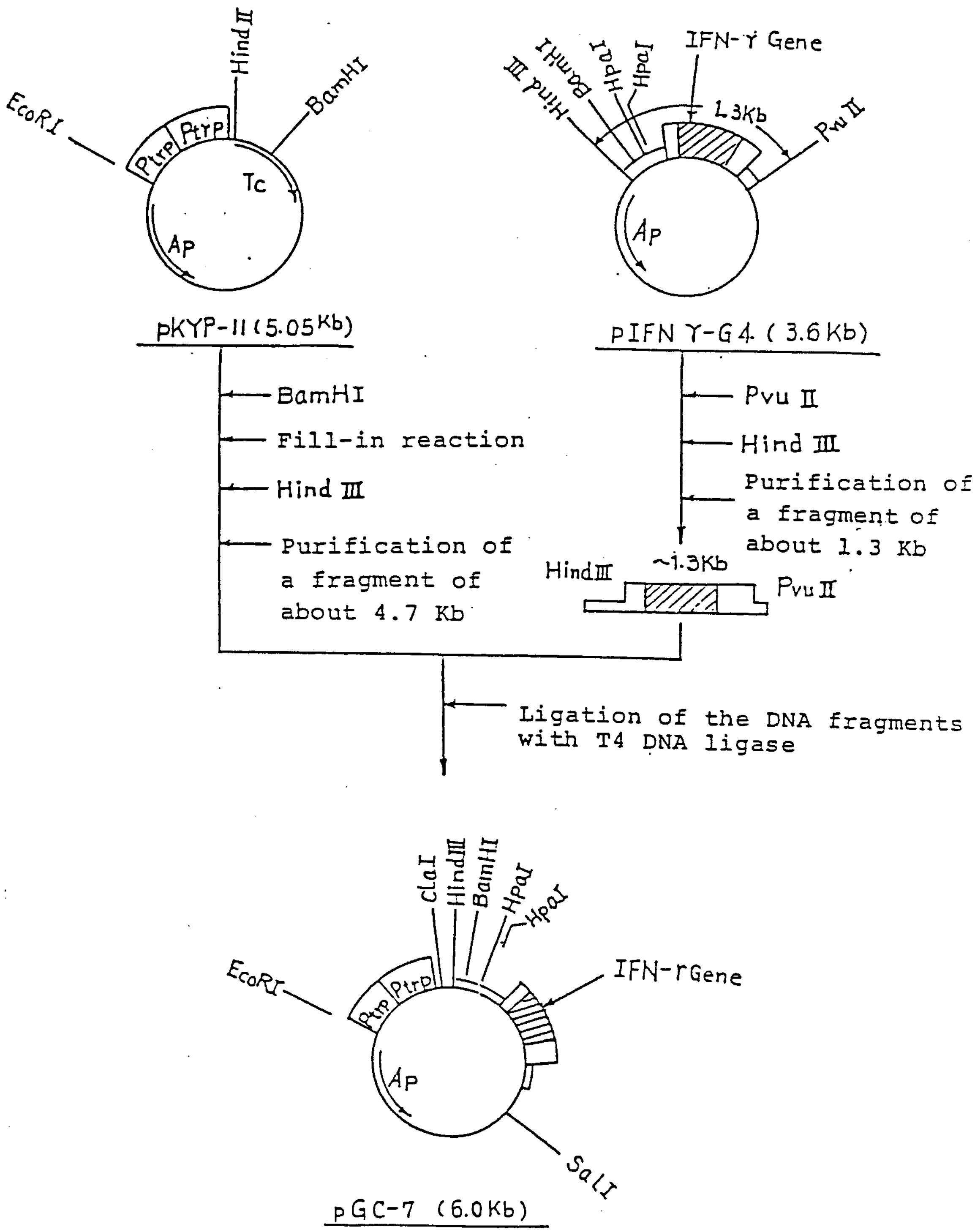
EcoRI
Ptrp
Ptrp
Hind III
BamHI
Tc
Ap
pKYP-11 (5.05Kb)
Hind III
BamHI
HpaI
HpaI
IFN-γ Gene
1.3Kb
Pvu II
Ap
pIFN γ-G4 (3.6Kb)
BamHI
Fill-in reaction
Hind III
Purification of
a fragment of
about 4.7 Kb
Pvu II
Hind III
Purification of
a fragment of
about 1.3 Kb
Hind III
~1.3Kb
Pvu II
Ligation of the DNA fragments
with T4 DNA ligase
claI
HindIII
BamHI
HpaI
HpaI
EcoRI
Ptrp
Ptrp
IFN-γ Gene
Ap
SalI
pGC-7 (6.0Kb)

F i g . 3

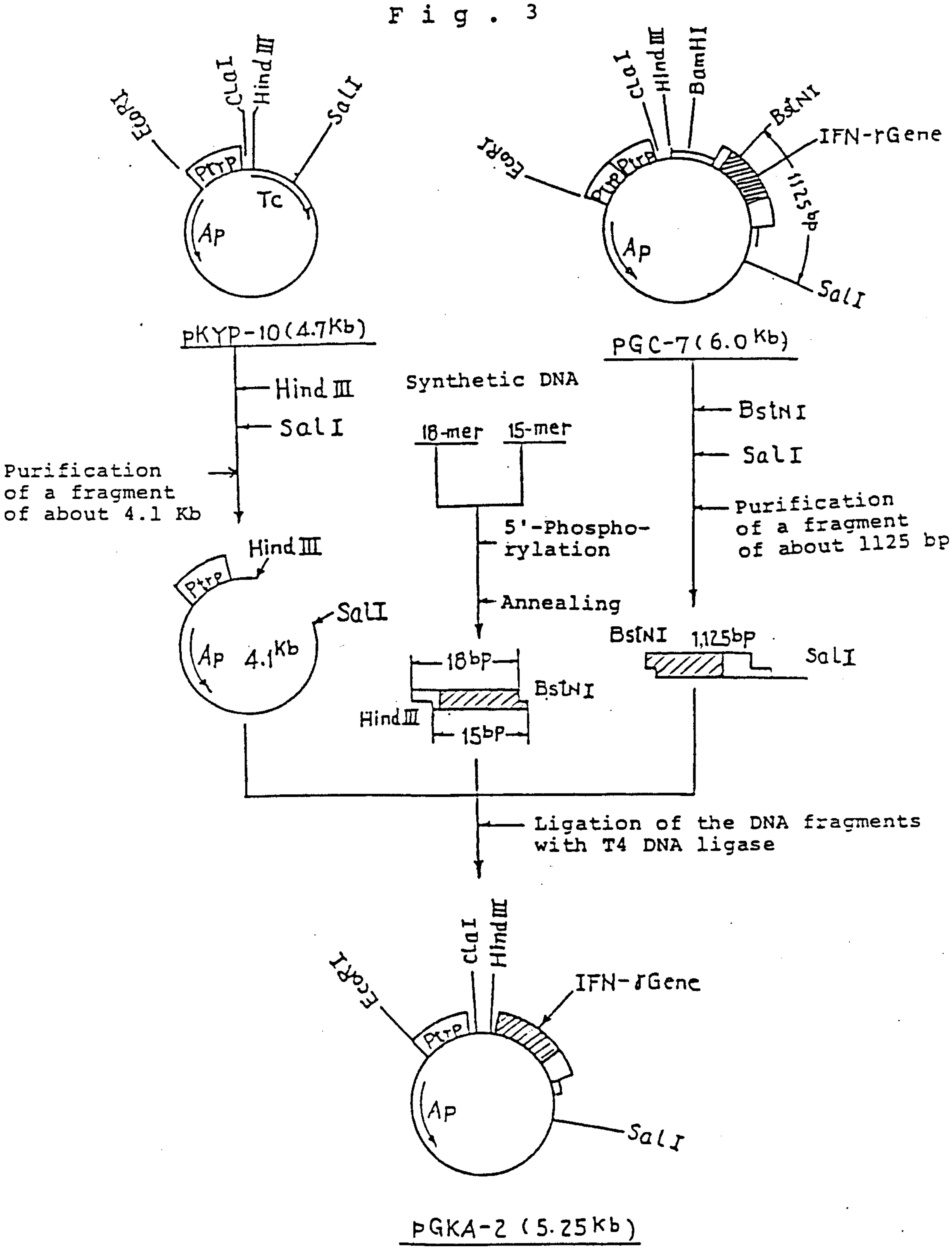
EcoRI
ClaI
HindIII
SalI
Ptrp
Tc
Ap
pKYP-10 (4.7Kb)
HindIII
BamHI
BstNI
IFN-γGene
1125bp
pGC-7 (6.0Kb)
Synthetic DNA
18-mer
15-mer
HindIII
SalI
Purification of a fragment of about 4.1 Kb
5'-Phosphorylation
Annealing
BstNI
SalI
Purification of a fragment of about 1125 bp
4.1Kb
18bp
15bp
1,125bp
Ligation of the DNA fragments with T4 DNA ligase
pGKA-2 (5.25Kb)

Fig. 4

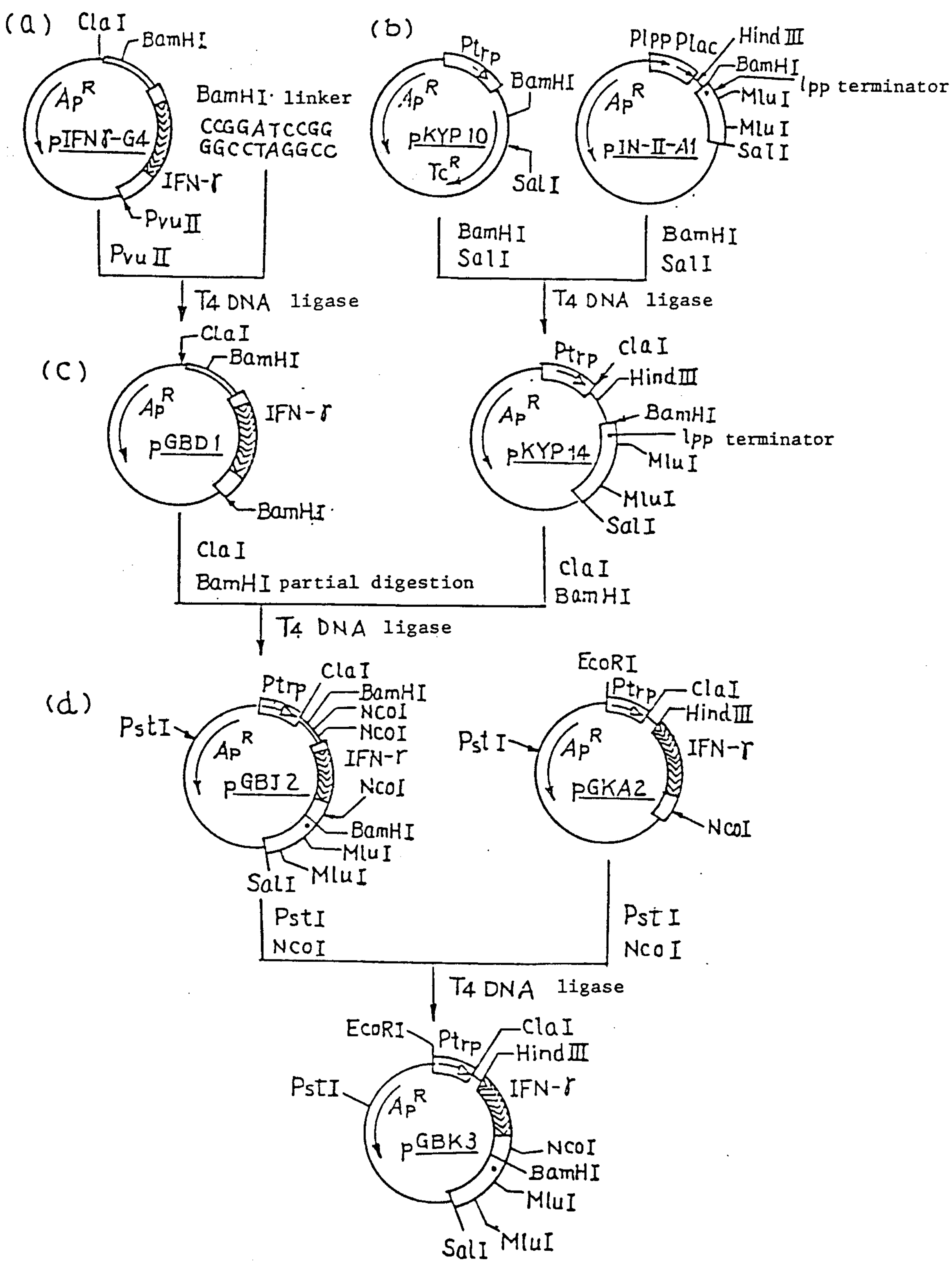
(a)
ClaI
BamHI
ApR
pIFNγ-G4
BamHI linker
CCGGATCCGG
GGCCTAGGCC
IFN-γ
PvuII
PvuII
(b)
Ptrp
BamHI
ApR
pKYP10
TcR
SalI
PlppPlac
HindIII
BamHI
lpp terminator
MluI
MluI
SalI
ApR
pIN-II-A1
BamHI
SalI
BamHI
SalI
T4 DNA ligase
T4 DNA ligase
(c)
ClaI
BamHI
ApR
pGBD1
IFN-γ
BamHI
Ptrp
ClaI
HindIII
BamHI
lpp terminator
MluI
MluI
SalI
ApR
pKYP14
ClaI
BamHI partial digestion
ClaI
BamHI
T4 DNA ligase
(d)
PstI
Ptrp
ClaI
BamHI
NcoI
NcoI
IFN-γ
NcoI
BamHI
MluI
MluI
SalI
ApR
pGBJ2
EcoRI
Ptrp
ClaI
HindIII
IFN-γ
PstI
ApR
pGKA2
NcoI
PstI
NcoI
PstI
NcoI
T4 DNA ligase
EcoRI
Ptrp
ClaI
HindIII
PstI
IFN-γ
ApR
pGBK3
NcoI
BamHI
MluI
SalI
MluI

Fig. 5

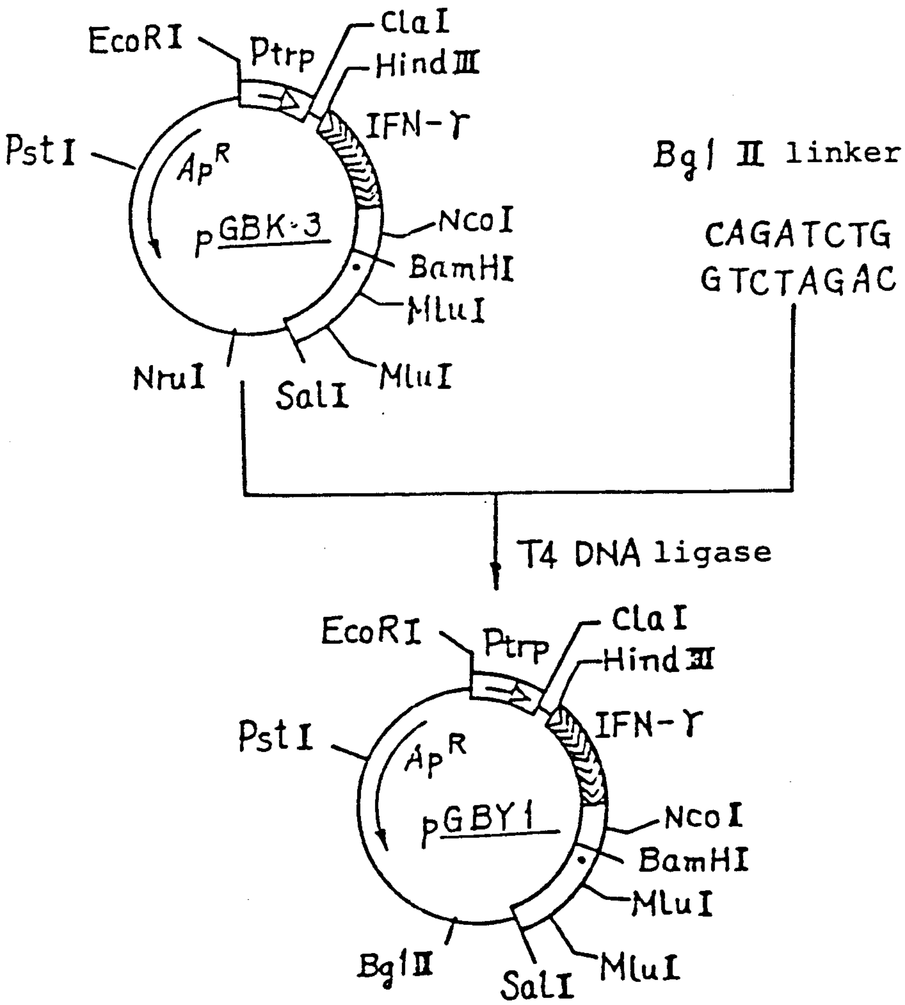
EcoRI
Ptrp
ClaI
HindIII
IFN-γ
PstI
Ap^R
pGBK-3
NcoI
BamHI
MluI
MluI
NruI
SalI
BglII linker
CAGATCTG
GTCTAGAC
T4 DNA ligase
EcoRI
Ptrp
ClaI
HindIII
IFN-γ
PstI
Ap^R
pGBY1
NcoI
BamHI
MluI
MluI
BglII
SalI